# EUROPEAN PATENT SPECIFICATION

(11) **EP 0 466 884 B1**
(45) Date of publication and mention of the grant of the patent: **22.05.1996**
(21) Application number: 91903410.8
(22) Date of filing: 07.02.1991
(51) Int. Cl.: A61M 1/00

(54) **JAR FOR PICKING UP AND RETAINING LIQUIDS**
GEFÄSS ZUM AUFNEHMEN UND BEWAHREN VON FLÜSSIGKEITEN
RECIPIENT DE RECUPERATION ET DE CONSERVATION DE LIQUIDES

(30) Priority: 07.02.1990 IT 1929490
(43) Date of publication of application: 22.01.1992
(73) Proprietor: FLOW-METER S.p.A., I-24100 Bergamo (IT)
(72) Inventor: PARACITO, Franco, I-24040 Levate (IT)
(74) Representative: Checcacci, Giorgio
(86) International application number: EP9100233
(87) International publication number: WO9112033

(56) References cited:
- GB-A- 1 318 620
- US-A- 3 768 478
- US-A- 4 111 204
- US-A- 4 681 571

## Description

### Technical Field

This invention relates to a jar for picking up and retaining liquids, being of a type which comprises a beaker, a closure cap fitting removably over said beaker, and connector elements formed integrally with the closure cap for connection to a respective suction conduit; for drawing up said liquids, and vacuum supply conduit, for establishing a vacuum within said jar.

The invention is particularly, but not exclusively, concerned with a jar intended for picking up organic liquids by suction, and reference will be made herein below to that field of application in the interest of a simplified description.

### Background Art

Well recognized in the medical and related environments is the need to have organic liquids drawn by suction effect from patients and/or invalids.

For this purpose, the prior art has proposed pick-up jars which include sealed lids and connector elements for connecting the jar to suction means of the vacuum source type.

The body of such jars consists in general of a beaker made of s sterilizable polycarbonate to which said lid is fitted removably in tight sealed relationship.

While being advantageous from various angles, and substantially achieving their objective, these prior pick-up jars have a major drawback in that they must be sterilized after each use.

This results in significant involvement for the operator and poses problems of sanitation and safety in use.

A prior art solution to avoid these drawbacks is disclosed in the US patent No. 4,111,204 which relates to a suction collection system embodying a disposable drainage receiving assembly including a flexible plastic bag.

A closure cap is fitted removably over a canister beaker and a disposable floppy bag container is sealed to the closure cap. First and second connector elements are formed integrally with the closure cap for connection to respective suction and vacuum supply conduits.

The first connector element is in fluid communication with the interior of the bag while the second connector element is in fluid communication with the interior of the canister and with the interior of the bag through a suction fixture defining a restricted fluid path.

These drawbacks reflect, moreover, in added time requirements to make such pick-up jars ready for a fresh working cycle.

The underlying-technical problem of this invention is to provide a jar for picking up and retaining organic liquids, which has such unique features of simple construction and operation, as well as low cost, as to overcome the aforementioned drawbacks with which the prior art is beset.

The solutive idea on which this invention stands is one of providing a pick-up jar of the disposable or single-use type.

### Disclosure of the Invention

Based on this solutive idea, the technical problem is solved by a closure cap for sealing off a liquid pick-up and retention jar as indicated and being characterized in that said second connector element is integrally formed with the cap and extends parallel to the plane of the cap, comprising a first cylindrical portion, extending parallel to the plane of the cap and jutting toward the interior of the bag meeting said valve holder, and a second independent cylindrical portion which is coaxial with the first portion and in fluid communication with the interior of the beaker through a side port, a sleeve being provided for a connection to the end of a conduit leading to a vacuum source, the sleeve being inserted into said second and first cylindrical portion and having at least a side port which is brought, by rotation of the sleeve, into registry with said side port of the second portion.

The feature and advantage of a cap according to the invention will become apparent from the following detailed description of an embodiment thereof, given by way of illustration and not limitation with reference to the accompanying drawings.

### Brief Description of Drawings

In the drawings:

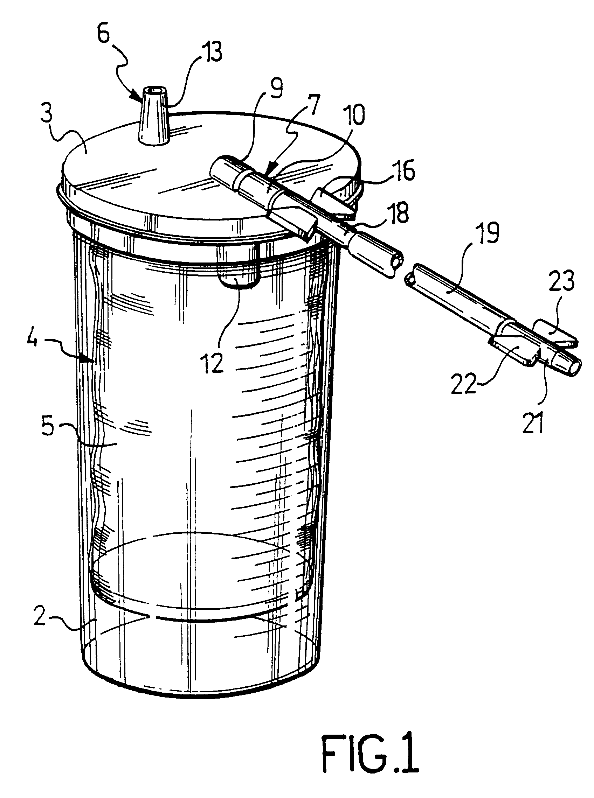

- Figure 1 is an elevation view showing in perspective a jar with a closure cap according to the invention;

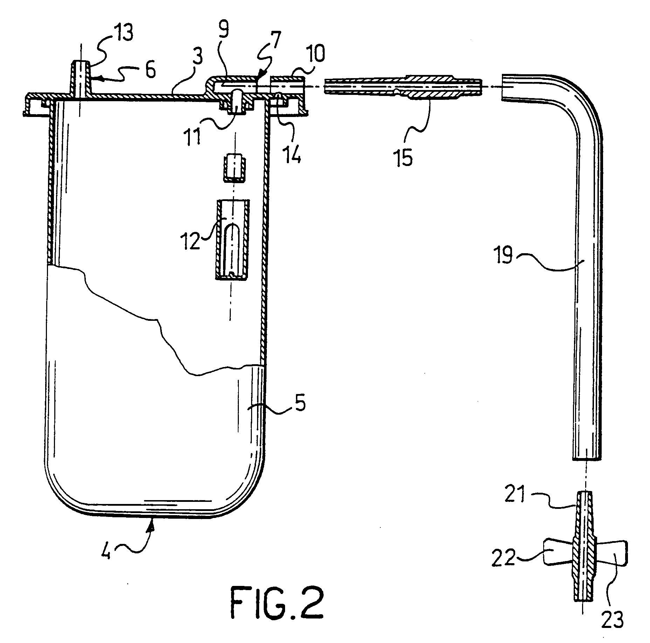

- Figures 2 and 7 are exploded views showing in vertical section a detail of the jar of Figure 1;
- Figures 3 to 6 are respective perspective views showing the jar of Figure 1 under different condictions of use.

### Best Mode of Carring Out the Invention

With reference to the drawing figures, generally shown at 1 is a pick-up jar for liquids intended, in particular but not limited thereto, for medical applications to pick up organic liquids by suction effect.

The jar 1 comprises a rigid beaker 2, made preferably of polycarbonate and provided with a closure cap 3.

Advantageously according to the invention, the jar 1 includes a disposable container 4 attached to the cap 3 and adapted to allow such liquids to be drawn up and retained therein.

Specifically, this container 4 is a bag collector 5 which is structurally independent of the beaker 2 and sealed to the cap 3. The bag 5 is made preferably of a soft and floppy synthetic plastics material.

Also formed integrally with the cap 3 are respective connector elements 6 and 7, each adapted for connection to a corresponding suction conduit, at one end, and to a vacuum supply conduit at the other end.

The former of said elements, i.e. the one denoted by the numeral 6, is basically a sleeve 13 formed integrally with the periphery of the cap 3 and jutting out perpendicularly thereto, at the remote end from the bag 5.

This sleeve 13 is in fluid communication with the interior of the bag 5 and adapted to receive the rubber end of a suction stylus, not shown because conventional.

The other connector element 7 comprises a first, T-like portion formed integrally with the cap 3 and having a first cylindrical section 9 which extends parallel to the plane of the cap and is closed at one end, and a second section 11 lying perpendicular thereto and jutting toward the interior of the bag 5.

Fitted to the end of said second section 11 is an overflow valve 12 effective to prevent the drawn liquids from leaking out of the bag 5.

Advantageously, the structure of the connector 7 is completed by a second cylindrical portion 10 formed in the cap 3 as a coaxial continuation of the section 9 of the first portion, over a predetermined distance from said section 9.

That second portion 10 has a side port 14 which puts it in fluid communication with the interior of the beaker 2 when the cap 3 is fitted tightly over the beaker 2.

The sections 9 and 10 are adapted to receive a connecting sleeve 15 which is passed snugly through the section 10 and has one end 18 formed integrally with two oppositely extending wings 16 and 17 for rotatively driving the sleeve.

Fitted to the end 18 of the connecting sleeve 15 is a conduit 19 for connection to the vacuum source which receives on its opposed end a further inlet connector consisting, in turn, of a sleeve 21 with conical opposed ends and being formed integrally with the oppositely extending wings 22 and 23.

The sleeve 15 is formed, moreover, with aligned side ports, set a predetermined distance apart, which are brought, by rotation of the sleeve, into registry with the section 11 of the connector 7 and the port 14, thereby allowing a vacuum to be established both inside the bag 5 and inside the beaker 2, sealed off by the cap 3.

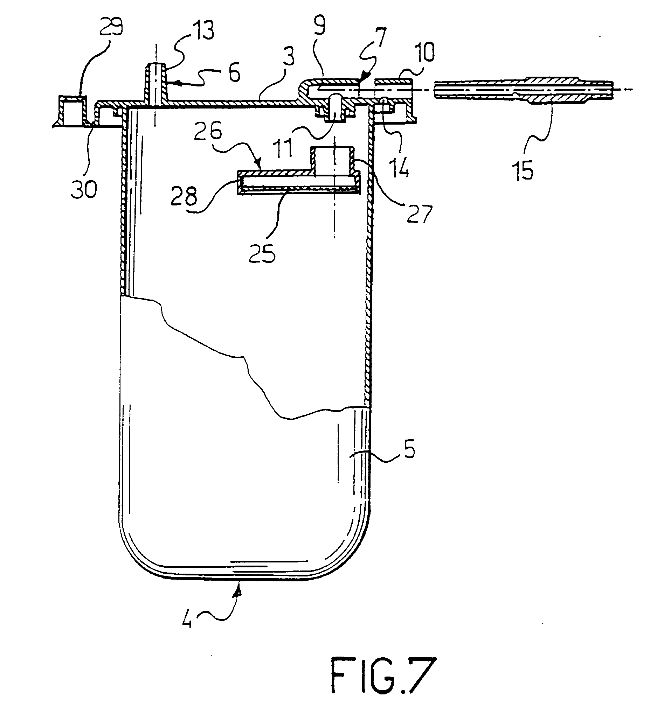

In a preferred embodiment, shown in Figure 7, the valve 12 comprises a water-repellent diaphragm 25 having micropores effective to only admit the suction flow to the jar interior.

This diaphragm 25 is mounted in a holder 26 made of a synthetic plastics material whereto it is sealed by a heated blade process.

The holder 26 comprises a first, sleeve-like portion 27 adapted to fit over the section 11 of the connector 7 and being formed without breaks integrally with an increased diameter portion 28 which accommodates the diaphragm 25.

Also provided is a stopper 29 associated with the cap 3 by a tearable link 30 and being adapted to fit over the sleeve 13 to shut off the inlet 6.

The operation of the pick-up jar according to the invention will be now described.

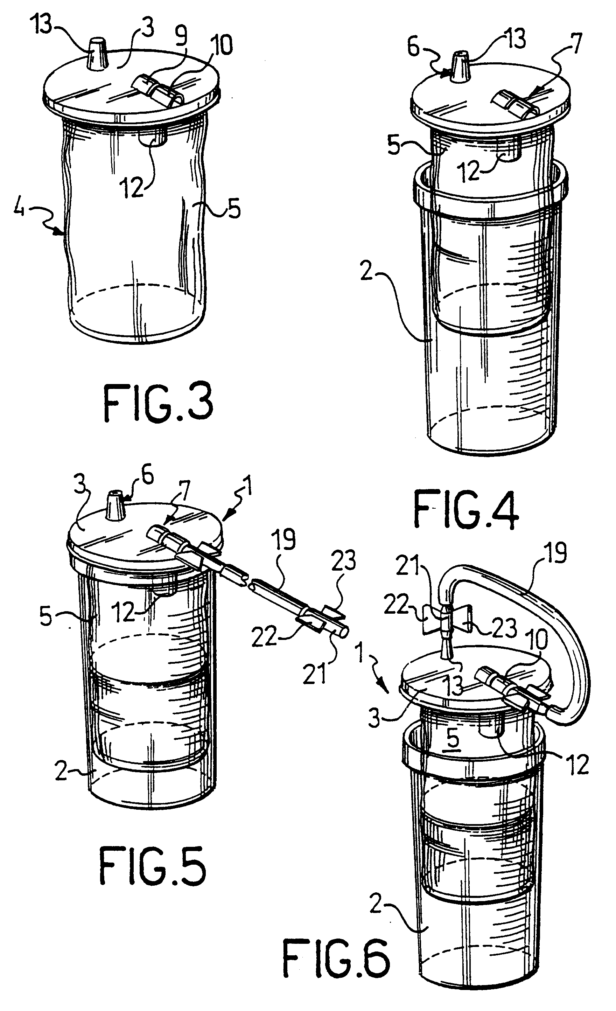

The cap 3, and the bag 5 made unitary therewith, is fitted tightly to the beaker 2, being careful to have the bag 5 arranged inside the beaker as shown in Figure 4.

The sleeve 15, as mounted on the end of the conduit 19 leading from the vacuum source, is inserted into the connector 7 on the cap 3 so as to enable a vacuum to be established both inside the bag 5 and in the interspace between it and the beaker 2 within the jar 1.

This is a necessary condition for proper operation of the device because, lacking the vacuum around the exterior of the bag 5 as well, the latter would collapse directly at the start of the suction operation.

In addition, the provision of the vacuum outside the bag 5 is favourable to the retention of the bag within the beaker 2 in the jar 1.

A suction stylus and respective conduit can now be connected to the other connector 6. During the suction operation, the valve 12 will prevent the drawn liquids from leaking out of the bag 5 into the jar 1 or possibly the suction conduit 19.

It will be appreciated that the functional features described hereinabove can provide for best sanitation and working safety of the inventive device.

On completion of the suction operation, the connection to the vacuum source can be cut off by turning the sleeve 15. Subsequently thereto, after disconnecting the suction stylus, on the one side, and the sleeve 21, on the other side, from the vacuum source, the container 4 can be sealed off by inserting the sleeve 21 to a push fit into the connector 6 as shown in Figure 6.

At this point, the disposable container 4 may be removed from the beaker 2, which will be therefore ready to accommodate a new container.

Where, on the other hand, the jar of this invention has the construction shown in Figure 7, the drawing action from the bag 5 may be protracted until the liquids being drawn up rise to the level of the water-repellent diaphragm 25. At this point, the micropores in the diaphragm are blocked and the suction stopped altogether.

It will be then sufficient that the stopper 29 be applied to the sleeve 13 in order for the bag collector 5 to become sealed off, and that the disposable container 4 be removed to make the beaker 2 ready to receive a new container.

The constructional peculiarity of having the cylindrical sections 9 and 10 of the connector 7 separated may be fully appreciated from the foregoing. This allows the solid portion of the cap 3 to be kept uniform in the annular region where the bag 5 is sealed, using a high frequency process.

On the whole, the pick-up jar of this invention exhibits uniquely simple constructional features which make it suitable for mass manufacture at a low cost. It also provides for sanitary and safe operating conditions which are specially valuable to the personnel detached to its use.

## Claims

1. A closure cap (3) for sealing off a liquid pick-up and retention jar (1) fitting removably over a beaker (2) and comprising a disposable floppy bag (5) container sealed to the closure cap (3), a first and a second connector elements (6, 7) formed integrally with the closure cap (3) for connection to respective suction and vacuum supply conduits, the first connector element (6) being in fluid communication with the interior of the bag (5), the second connector element (7) being in fluid communication with the interior of beaker (2) and with the interior of the bag (5) through a holder (26) of an overflow valve (12), characterized in that said second connector element (7) is integrally formed with the cap (3) and extends parallel to the plane of the cap comprising a first cylindrical portion (9), extending parallel to the plane of the cap (3) and jutting toward the interior of the bag (5) meeting said valve holder (26), and a second independent cylindrical portion (10) which is coaxial with the first portion (9) and in fluid communication with the interior of the beaker (2) through a side port (14), a sleeve (15) being provided for a connection to the end of a conduit (19) leading to a vacuum source, the sleeve (15) being inserted into said second (10) and first cylindrical portion (9) and having at least a side port which is brought, by rotation of the sleeve, into registry with said side port (14) of the second portion (10).

2. A closure cap according to claim 1, characterized in that a couple of oppositely extending wings (16, 17) are integrally formed with said sleeve (15) for rotating the same.

3. A closures cap according to claim 1, characterized in that it comprises a second sleeve (21) connected to the other end of said conduit (19) and pushed fit into said first connector element (6) sealing the openings the said cap (3) when the suction is completed.

4. A closure cap according to claim 1, characterized in that said overflow valve (12) comprises essentially a water-repellant diaphragm mounted in said holder (26).

5. A jar for picking up and retaining liquids comprising: a beaker (2) and a closure cap (3) according to claim 1.

## Patentansprüche

1. Eine Verschlußkappe (3) zum dichten Verschließen eines Flüssigkeits-Aufnahme- und Speichergefäßes (1), wobei die Verschlußkappe (3) abnehmbar auf einen Becher (2) paßt und einen Einwegbehälter in Form eines flexiblen Beutels (5) aufweist, der mit der Verschlußkappe (3) verschlossen ist, wobei diese Verschlußkappe (3) ein erstes Anschlußelement (6) und ein zweites Anschlußelement (7) aufweist, wobei die Anschlußelemente (6, 7) mit der Verschlußkappe (3) einstückig ausgebildet sind und zum Anschließen an jeweilige Saug- und Vakuumversorgungsleitungen dienen, wobei das erste Anschlußelement (6) in Fluidverbindung mit dem Inneren des Beutels (5) steht, und wobei das zweite Anschlußelement (7) in Fluidverbindung mit dem Inneren des Bechers (2) und mit dein Inneren des Beutels (5) durch einen Halter (26) eines Überlaufventils (12) steht,
**dadurch gekennzeichnet,**
daß das zweite Anschlußelement (7) mit der Kappe (3) einstückig ausgebildet ist und sich parallel zu der Ebene der Kappe erstreckt und einen ersten zylindrischen Bereich (9) aufweist, der sich parallel zu der Ebene der Kappe (3) erstreckt und zu dem Inneren des Beutels (5) hin vorsteht und mit dem Ventilhalter (26) zusammentrifft, wobei das zweite Anschlußelement (7) einen zweiten unabhängigen zylindrischen Bereich (10) aufweist, der mit dem ersten Bereich (9) koaxial ist und in Fluidverbindung mit dem Inneren des Bechers (2) durch eine seitliche Öffnung (14) steht, wobei eine Hülse (15) für eine Verbindung mit dem Ende einer Leitung (19) vorgesehen ist, die zu einer Vakuumquelle führt, und wobei die Hülse (15) in den zweiten (10) und in den ersten zylindrischen Bereich (9) eingesetzt ist und wenigstens eine seitliche Öffnung aufweist, die durch Drehen der Hülse in Deckung mit der seitlichen Öffnung (14) des zweiten Bereiches (10) gebracht ist.

2. Eine Verschlußkappe gemäß Anspruch 1,
**dadurch gekennzeichnet,**
daß ein Paar von sich entgegengesetzt zueinander erstreckenden Flügeln (16, 17) vorgesehen ist, welche mit der genannten Hülse (15) zum Drehen derselben einstückig ausgebildet sind.

3. Eine Verschlußkappe gemäß Anspruch 1,
**dadurch gekennzeichnet,**
daß sie eine zweite Hülse (21) aufweist, die mit dem anderen Ende der genannten Leitung (19) verbunden ist und in das genannte erste Anschlußelement (6) passend gedrückt ist, wobei die zweite Hülse (21) die Öffnungen der genannten Kappe (3) verschließt, wenn der Saugvorgang vervollständigt wird.

4. Eine Verschlußkappe gemäß Anspruch 1,
**dadurch gekennzeichnet,**
daß das Überlaufventil (12) im wesentlichen eine wasserabweisende Membrane aufweist, die in dem genannten Halter (26) eingebaut ist.

5. Ein Gefäß zum Aufnehmen und Aufbewahren von Flüssigkeiten, wobei das Gefäß aufweist: einen Becher (2) und eine Verschlußkappe (3) gemäß Anspruch 1.

## Revendications

1. Un couvercle de fermeture (3) pour fermer de façon étanche un récipient de récupération et de conservation de liquides, s'adaptant de façon amovible sur un bocal (2) et comprenant un conteneur sous la forme d'un sac souple à usage unique (5) scellé au couvercle de fermeture (3), des premier et second éléments de raccordement (6, 7) faisant partie intégrante du couvercle de fermeture (3) pour établir des raccordements à des conduits respectifs d'aspiration et de mise en dépression, le premier élément de raccordement (6) étant en communication fluidique avec l'intérieur du sac (5), le second élément de raccordement (7) étant en communication fluidique avec l'intérieur du bocal (2) et avec l'intérieur du sac (5) par l'intermédiaire d'un support pour une valve d'arrêt (12), caractérisé en ce que ledit second élément de raccordement (7) fait partie intégrante du couvercle (3), s'étend parallèlement au plan du couvercle et comprend une première partie cylindrique (9), s'étendant parallèlement au plan du couvercle (3) et faisant saillie vers l'intérieur du sac (5) jusqu'à la rencontre du support de valve (26), et une seconde partie cylindrique indépendante (10) qui est coaxiale avec la première partie (9) et en communication fluidique avec l'intérieur du bocal (2) par l'intermédiaire d'une ouverture (14) dans sa paroi, un manchon (15) étant prévu pour le raccordement à l'extrémité d'un conduit (19) menant à une source de vide, le manchon (15) étant inséré dans lesdites seconde et première parties cylindriques (10, 9) et présentant au moins une ouverture dans sa paroi qui est amenée, par rotation du manchon, en correspondance avec ladite ouverture (14) dans la paroi de la seconde partie (10).

2. Un couvercle de fermeture selon la revendication 1, caractérisé en ce qu'une paire d'oreilles opposées font partie intégrante dudit manchon (15), qui servent pour le faire tourner.

3. Un couvercle de fermeture selon la revendication 1, caractérisé en ce qu'il comprend un second manchon (21) raccordé à l'autre extrémité dudit conduit (19) et s'enfonçant dans ledit premier élément de raccordement (6) pour fermer les ouvertures dudit couvercle (3) lorsque l'aspiration est achevée.

4. Un couvercle de fermeture selon la revendication 1, caractérisé en ce que ladite valve d'arrêt (12) comprend essentiellement une membrane imperméable à l'eau montée dans ledit support (26).

5. Un récipient pour récupérer et conserver des liquides comprenant : un bocal (2) et un couvercle de fermeture (3) selon la revendication 1.
